# FASCICULE DE BREVET EUROPEEN

(11) **EP 2 309 954 B1**
(45) Date de publication et mention de la délivrance du brevet: **29.10.2014**
(21) Numéro de dépôt: 08786642.2
(22) Date de dépôt: 30.07.2008
(51) Int. Cl.: A61F 7/00, A61H 33/06, A61F 7/02

(54) **EQUIPEMENT POUR LE TRAITEMENT CORPOREL FORMÉ PAR UNE ENVELOPPE EN AU MOINS DEUX PARTIES COMPLEMENTAIRES**
GERÄT ZUR BEHANDLUNG DES KÖRPERS MIT EINEM GEHÄUSE AUS MINDESTENS ZWEI KOMPLEMENTÄREN TEILEN
APPARATUS FOR BODY TREATMENT CONSISTING OF A SHELL MADE OF AT LEAST TWO COMPLEMENTARY PORTIONS

(43) Date de publication de la demande: 20.04.2011
(73) Titulaire: Dufay, François, 39300 Loulle (FR)
(72) Inventeur: Dufay, François, 39300 Loulle (FR)
(74) Mandataire: Decamps, Alain René François
(86) Numéro de dépôt international: PCT/EP2008/060013
(87) Numéro de publication internationale: WO 2010/012302

(56) Documents cités:
- EP-A- 0 325 771
- EP-A- 1 170 033
- WO-A-01/93790
- FR-A- 2 544 202
- GB-A- 1 352 142
- US-A- 5 038 769

## Description

### DOMAINE DE L'INVENTION

La présente invention concerne le domaine du traitement corporel, topique ou systémique, curatif ou préventif, dans les domaines thérapeutique, esthétique et cosmétique pour l'homme et l'animal et plus particulièrement le traitement de plaies ouvertes.

### ARRIERE PLAN DE L'INVENTION

Le traitement habituel de plaies ouvertes consiste à apposer des pansements sur la plaie. Le problème qui se pose est celui du contact entre le pansement et la plaie, et les risques de prolifération microbienne.

Afin de palier à cet inconvénient, plusieurs variantes de dispositifs de traitement d'une plaie ou d'une quelconque blessure ou maladie d'une partie du corps d'un patient par exposition de cette partie à un fluide ont été proposées. De façon générale, ces dispositifs comprennent au moins une paroi formant une enceinte ou une cavité scellée de manière plus ou moins hermétique autour de la partie du corps à traiter et comprenant des moyens d'injection d'un fluide de traitement dans l'enceinte pour sa mise en contact avec ladite partie du corps à traiter et des moyens d'évacuation de fluides de cette enceinte. Dans ce domaine, on peut citer comme exemples les brevets US4003371, EP0008719, US4432354, US4447504 et EP1089698.

Dans le domaine du traitement des brûlures et hypothermies, on peut citer des dispositifs semblables à ceux mentionnées plus hauts, tels que WO0193790 ou US5000164, le principe général du dispositif étant identique, seuls les fluides utilisés, les pression, température, pH, etc. dans l'enceinte variant selon le type de traitement et selon la partie du corps à traiter. Bien entendu la forme du dispositif varie également selon que la partie du corps à traiter est une jambe, un bras, un cou, un torse, etc.

GB1352142 divulgue un equipement selon la preambule de la revendication 1 qui suit.

Quelque soit le type de traitement à appliquer à une partie du corps, le contrôle de l'atmosphère régnant à l'intérieur de l'enceinte est très important. En particulier, la température doit être contrôlée car la fenêtre de températures du traitement est généralement étroite dû aux faits, d'une part, que le corps, surtout si blessé, ne peut être exposé longtemps à de grandes plages de températures et, d'autre part, les principes actifs utilisés (substances thérapeutiques, huiles essentielles, etc.) sont également sensibles à la température et peuvent se dégrader ou perdre de leur efficacité avec la température. De plus, dans les domaines thérapeutiques, il est important de contrôler la température afin de limiter, voire annuler la prolifération bactérienne aérobie ou anaérobie. L'avantage de dispositifs tels que décrits plus haut étant justement qu'ils peuvent être utilisés à peu près n'importe où et à tout moment, la température ambiante autour de l'enceinte peut varier énormément d'un traitement à l'autre, ce qui peut avoir des conséquences néfastes sur la reproductibilité du traitement. Il est clair qu'en augmentant le flux du fluide à travers l'enceinte, la température absolue peut être relativement contrôlée, mais au prix d'un abaissement de la température ressentie par la personne traitée qui peut devenir inconfortable. De plus, selon le principe actif utilisé cette solution peut résulter très coûteuse.

Aucun des dispositifs décrits plus haut n'aborde ce problème de contrôle de la température dans l'enceinte. Il demeure donc dans l'art un problème à résoudre.

### RESUME DE L'INVENTION

La présente invention est définie dans la revendication indépendante qui suive. Des variantes préférées sont définies dans les revendications dépendantes. L'objet de la présente invention est de proposer un équipement de traitement amélioré permettant d'apporter des principes actifs à la surface d'une partie du corps humain ou animal, en préservant au maximum de tout contact la partie à traiter et offrant un meilleur contrôle des conditions régnant autour de la partie traitée, en particulier les conditions thermiques. Elle a pour but de maintenir un climat propice au traitement appliqué, par exemple la cicatrisation d'une plaie, en évitant des gradients de température importants autour de la partie traitée. Un traitement selon l'invention est plus indolore car il évite le contact de la plaie avec des exsudats produits par la plaie, et assure un environnement favorable à la cicatrisation.

Selon l'invention, il est essentiel que l'enveloppe soit constituée par au moins deux parties complémentaires en un matériau isolant thermiquement.

De préférence, l'équipement comporte deux coques complémentaires de forme générale sensiblement cylindrique, tronconique ou prismatique. L'enveloppe peut présenter une première extrémité frontale ouverte et une seconde extrémité frontale fermée ou les deux extrémités ouvertes, les extrémités ouvertes pouvant comprendre un ou plusieurs orifices chacun. Dans le cas où il y a plusieurs orifices dans une extrémité, ceux-ci peuvent communiquer avec une enceinte unique ou, au contraire, communiquer avec plusieurs compartiments distincts correspondants, inclus dans l'enveloppe. Les différentes variantes de géométries de l'enveloppe, et du nombre de compartiments dans l'enveloppe dépendent de la partie du corps à traiter et du type de traitement à appliquer.

Afin de préserver au mieux les conditions régnant à l'intérieur de la cavité formée par l'enveloppe, en particulier du point de vue température, l'enveloppe est composée de matériaux permettant de réduire la conduction thermique moyenne de celle-ce à moins de 65 mW / m·K mesurée selon EN-12667, de préférence à moins de 45 mW / m·K, voire 40 mW / m·K.

Pour atteindre de telles valeurs d'isolation thermique, l'enveloppe est avantageusement constituée de matériaux cellulaires imperméables, par exemple un polypropylène expansé (PPE), un polystyrène expansé (PSE), du polyuréthane expansé (PUE) ou tout autre matériau expansé présentant les propriétés thermiques, structurelles et de compatibilité requises par l'application à laquelle est destinée l'équipement de la présente invention. Par exemple un PPE de masse volumique comprise entre 20 et 60 g/l a typiquement une conductivité thermique de l'ordre de 38 à 42 mW / m·K, et un PSE de masse volumique comprise entre 19 et 30 g/l a typiquement une conductivité thermique de l'ordre de 36 à 39 mW / m·K.

Selon l'invention ledit matériau présente un traitement de lissage de la surface pour en faciliter le nettoyage par un traitement thermique (cuisson ou glaçage) ou par l'application de films plaqués et drapés formant une peau sur les surfaces internes et/ou externes du matériau.

De manière alternative, ou en combinaison avec des matériaux cellulaires, des matériaux fibrillaires peuvent aussi être utilisés, tels que des fibres minérales (par exemple, des fibres de verre, ou de roche) ou polymériques (par exemple, polyéthylène, polypropylène). La laine de verre a typiquement une conductivité thermique de l'ordre de 34 à 65 mW / m·K, alors que la laine de roche tourne autour de 38 à 47 mW / m·K.

Pour des raisons structurelles, d'étanchéité aux fluides et d'hygiènes le matériau fibrillaire est généralement compris entre deux peaux pour former un sandwich. Les peaux peuvent être souples ou rigides selon le type d'application. De préférence l'enveloppe est structurellement autoportante et donc des peaux rigides sont préférées. Si l'isolation thermique est suffisante, les fibres peuvent être imprégnées de résine pour former un matériau composite.

Avantageusement, l'enveloppe présente en outre, au niveau de chaque ouverture frontale, un moyen de raccordement avec la surface du membre. De préférence, ledit moyen de raccordement est constitué par un manchon apte à être raccordé à l'une de ses extrémités à la périphérie d'une ouverture de l'enveloppe, et à l'autre extrémité venir en contact avec le corps. Selon une variante, ledit moyen de raccordement est constitué par un anneau gonflable coopérant avec la surface de ladite enveloppe. Un tel moyen de raccordement -ou tout autre moyen équivalent-permet de mieux maintenir les conditions thermiques dans la cavité formée par l'enveloppe.

Selon un mode de réalisation particulier, l'une au moins des parties présente des moyens de soutènement de la partie du corps entourée par l'enveloppe. Par exemple, ledit moyen de soutènement est constitué de sangles transversales. Selon une alternative, l'une des coques formant l'enveloppe présente des plots d'ancrage desdites sangles transversales, lesdits plots d'ancrage étant de préférence sécables ou démontables. Avantageusement, lesdits plots d'ancrage sont constitués par des parties saillantes prévues sur la tranche de la coque, et en ce que la coque complémentaire présente des évidements complémentaires à ces parties saillantes.

Selon une autre variante, ledit moyen de soutènement est constitué par des berceaux transversaux. Selon une variante, ledit moyen de soutènement est constitué par des coussins coopérant avec le fond de l'une des coques.

Selon un mode de réalisation particulier, l'enveloppe est constituée par deux coques articulées ou non par une charnière. Par exemple, l'enveloppe peut être constituée par deux coques indépendantes, l'une des coques présentant un moyen de réception de l'autre coque en position ouverte. Avantageusement, l'une des coques présente sur sa tranche au moins une nervure complémentaire avec au moins une cannelure prévue sur la coque complémentaire, pour créer une chicane.

De préférence, l'une des coques présente une gorge pour le maintien d'une conduite de gaz. Selon un mode de réalisation particulier, au moins une pièce de raccordement d'une conduite de gaz de l'équipement est formée par une platine prolongée par un embout tubulaire, ladite platine coopérant avec un logement complémentaire prévu dans l'enveloppe.

L'équipement selon l'invention comporte un système d'alimentation d'au moins un principe actif, ce dernier pouvant être sous forme gazeuse, liquide, ou solide (particulaire) et avantageusement transporté par un gaz porteur. Selon une variante, il comporte un système d'alimentation en gaz comprenant un moyen d'enrichissement d'un gaz porteur avec au moins un principe actif. Selon une autre variante, il comporte en outre un système d'alimentation en gaz comprenant une enceinte de nébulisation pour enrichir en principe actif un gaz porteur.

Selon une autre variante, il comporte en outre un système d'alimentation en gaz comprenant un mélangeur pour enrichir un gaz porteur avec au moins un principe actif liquide ou solide conditionné dans un contenant raccordé au système d'alimentation.

Selon un mode de réalisation particulier, il comporte en outre au moins un réservoir d'alimentation en gaz. De préférence, le système d'alimentation en gaz comprend des moyens de régulation de l'hygrométrie et/ou de la température et/ou du PH et/ou du débit et/ou des caractéristiques physico-chimiques du gaz injecté.

Avantageusement, ledit système d'alimentation en gaz comprend des moyens de traitement des gaz à la sortie de l'enveloppe. Selon un exemple de réalisation, lesdits moyens de traitement des gaz comprennent un barboteur, relié à une source de vide. Selon une variante, il comporte en outre un système de raccordement de l'entrée de l'enveloppe à une source de vide pour établir dans l'enveloppe une pression inférieure à la pression atmosphérique.

De préférence, l'enveloppe présente au moins une fenêtre transparente.

Selon un mode de réalisation particulier, l'enveloppe présente une ouverture prolongée vers l'intérieur par un manchon pour permettre des manipulations à l'intérieur de l'enceinte formée par les deux coques. Selon une autre variante, il comporte au moins un capteur physico-chimique pour l'analyse de l'atmosphère dans l'enceinte formée par les deux coques. De préférence, l'équipement comporte un moyen de verrouillage des deux coques. Avantageusement, l'une au moins des coques incorpore une étiquette radio-fréquence.

### Brève description des Figures

L'invention sera mieux comprise à la lecture de la description qui suit, se référant aux dessins annexés où :
- la figure 1 représente une vue en perspective d'un exemple de réalisation d'un équipement selon l'invention ;
- les figures 2a et 2b représentent des vues de la coque inférieure selon un plan de coupe transversal avec un détail d'une paroi comprenant un coeur en matériau cellulaire pris en sandwich entre deux peaux;
- la figure 3 représente une vue de l'intérieur de la coque inférieure ;
- les figures 4a et 4b représentent deux vues de l'équipement en position ouverte ;
- la figure 5 représente une vue de détail de la liaison entre les deux coques ;
- la figure 6 représente une vue en détail de l'extrémité ouverte de l'équipement ;
- la figure 7 représente une vue de détail du raccordement entre l'entrée de fluide et la coque ;
- la figure 8 représente une vue en coupe de l'extrémité fermée de l'équipement ;
- les figures 9 et 10 représentent des vues d'une variante de réalisation ;
- la figure 11 représente une vue d'une autre variante de réalisation.

### Description détaillée de l'invention

L'invention sera décrite dans ce qui suit en référence à un exemple non limitatif de réalisation.

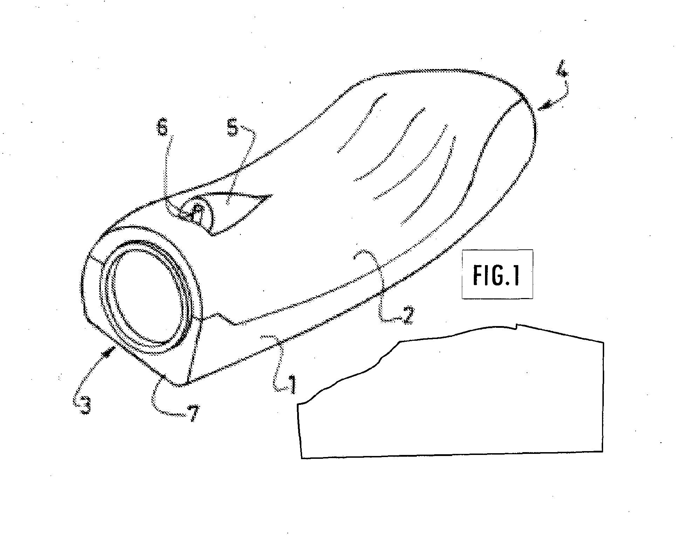

La figure 1 représente une vue en perspective d'un exemple de réalisation d'un équipement selon l'invention. Il se présente sous la forme d'une enveloppe rigide constituée de deux coques (1, 2) raccordées entre elles pour former une enceinte à l'intérieur de laquelle peut être maintenue une partie du corps, par exemple la jambe ou un bras. Les coques (1, 2) présentent dans l'exemple décrit une forme allongée, avec une première extrémité frontale (3) ouverte et une seconde extrémité frontale opposée (4) fermée. La coque supérieure (2) présente une partie saillante (5) s'ouvrant sur une entrée de gaz (6) communiquant avec l'intérieur de l'enceinte délimitée par les coques (1, 2). Une cannelure (7) prévue à la surface inférieure de la coque (1) est destinée à loger le tuyau d'alimentation en gaz.

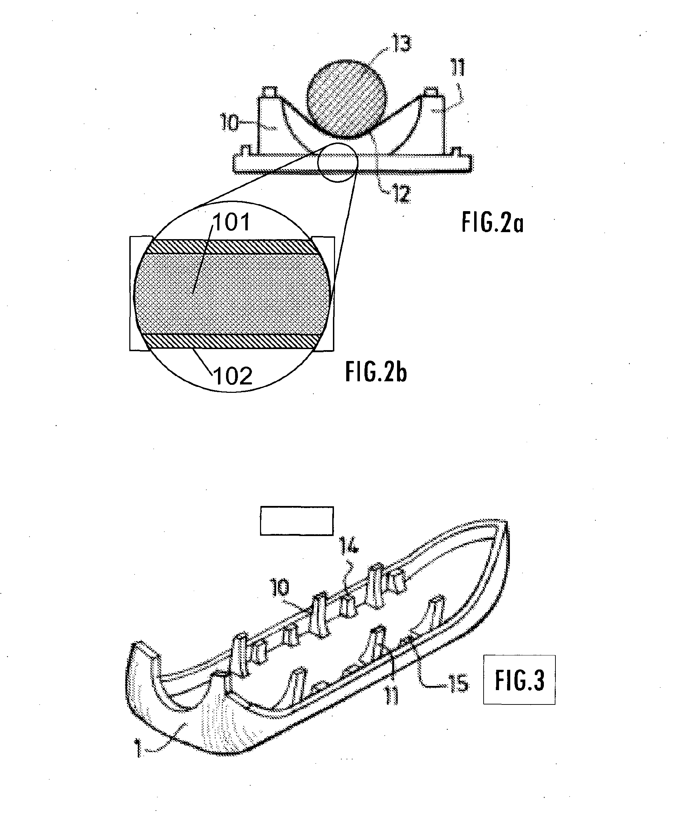

Tel qu'illustré sur la Figure 2b, les deux coques (1, 2) peuvent être constituées en un matériau expansé (101), par exemple du polypropylène expansé ou du polystyrène expansé. Elles peuvent avoir subi un traitement de surface (102) permettant de limiter la diffusion gazeuse à travers la paroi, de garantir une certaine hygiène, de renforcer structurellement le système, et/ou conférer un aspect esthétique agréable. Les traitements de surface possible sont par exemple (a) le glaçage, par lequel on lisse la surface du matériau cellulaire en appliquant un traitement thermique et, optionnellement, un traitement mécanique à la surface ou (b) l'application d'une peau sur une ou les deux faces des parois des coques. Les matériaux pour les peaux varient selon la ou les fonctions de celles-ci : barrière, hygiène, structurelle, ou esthétique.

Par exemple, pour des effets barrière aux gaz, des films mutlicouches dont les couches extérieures ont une fonction d'adhésion au support et de soudabilité alors que la ou les couches intérieures ont une fonction proprement de barrière aux gaz. En utilisant des films du type PE/EVOH/PE ou PS/EVPH/PS ou similaires on peut obtenir des valeurs de perméabilité aux gaz très faibles. On peut aussi envisager l'utilisation de feuilles métalliques, telles des feuilles d'aluminium. L'hygiène du système sera facilitée par l'application de peaux lisses et peu adhérentes, telles certains polymères à basse énergie de surface tels le polyéthylène (PE, HDPE), polypropylène (PP), polyoxométhylène (POM), polytetrafluorethylène (PTFE), etc. Pour des applications structurelles des matériaux plus rigides, tels des métaux, polymères structurels (epoxy, polyesters, etc.), composites renforcés de fibres, etc. peuvent faire l'affaire. Enfin pour l'aspect esthétique, une apparence de cuir, ou toute autre texturation de la surface extérieure peuvent être impartis par des techniques bien connues par l'homme du métier, ou au contraire une surface lisse peut également être obtenue par des techniques développées par exemple dans l'industrie automobile ou d'équipements sportifs. Tant que l'isolation thermique moyenne de l'enveloppe demeure suffisante, une des coques peut présenter une fenêtre transparente permettant d'observer l'évolution de la plaie, et d'appliquer des traitements par rayonnements. Un système de double vitrage peut être utilisé afin de réduire la conduction thermique de la fenêtre et ainsi permettre d'avoir une fenêtre de plus grandes dimensions sans perdre en propriétés thermiques et en réduisant les gradients thermiques.

Dans le cas d'application de peaux à la surface du matériau isolant, des considérations d'adhésion desdites peaux au matériau isolant doivent être considérées. En particulier, il peut résulter difficile de trouver des matériaux adhérant convenablement à des matériaux tels que le polyptopylène qu'il soit expansé ou sous forme de fibres. Dans ce cas il peut être avantageux d'utiliser un matériau pour les peaux qui soit compatible, voire de même nature que le matériau constituant le coeur de la paroi. Dans le cas du polypropylène expansé, par exemple, l'application de peaux en polypropylène est particulièrement préférée.

Il est clair qu'un traitement de surface des matériaux cellulaires ou fibrillaires constituant l'enveloppe de l'équipement de la présente invention devrait avantageusement assurer une amélioration des propriétés de barrière aux gaz, car une trop grande perméabilité aux gaz des parois entraînerait, d'une part, une consommation trop importante de gaz et, surtout, de principe actif et, d'autre part, un transfert de chaleur qui annulerait les effets recherchés par l'utilisation de ces matériaux isolants thermiquement. En effet, un matériau tel que le polypropylène expansé offre généralement une perméabilité, mesurée selon ASTM F1927-98, de l'ordre de 1000 à 10.000 cm³ / m².atm.24h selon la masse volumique et l'épaisseur du matériau, ce qui est insuffisant pour la plupart des applications visées par la présente invention. Cette perméabilité peut être réduite par l'application de peaux en polypropylène par exemple, jusqu'à des valeurs inférieures à 600 et même 300 cm³ / m².atm.24h selon le grade et l'épaisseur du film. Des valeurs inférieures à 200, 100 et dans le cas de matériaux barrières spécifiques, bien connus de l'homme du métier, inférieures à 10 et pouvant atteindre 1 cm³ / m².atm.24h peuvent être atteintes.

La figure 2a représente une vue de la coque inférieure selon un plan de coupe transversal. La coque inférieure (1) présente des poteaux latéraux (10, 11) se dressant perpendiculairement au fond. Ces poteaux soutiennent des sangles (12) s'étendant transversalement, pour assurer le soutien du membre (13) qui est enfermé entre les deux coques (1, 2).

La figure 3 représente une vue de l'intérieur de la coque inférieure. La coque inférieure (1) présente l'allure générale d'une barque à fond plat. Les poteaux sont répartis sur une partie de la longueur de la coque, de part et d'autre de la partie centrale. Les plots sont sécables ou amovibles, et ceux qui ne servent pas au soutien d'une sangle transversale sont coupés ou démontés (14, 15). La sangle présente à chaque extrémité des séries de trous destinés à recevoir l'extrémité tubulaire des plots, et permettant le réglage de la tension et du positionnement du membre.

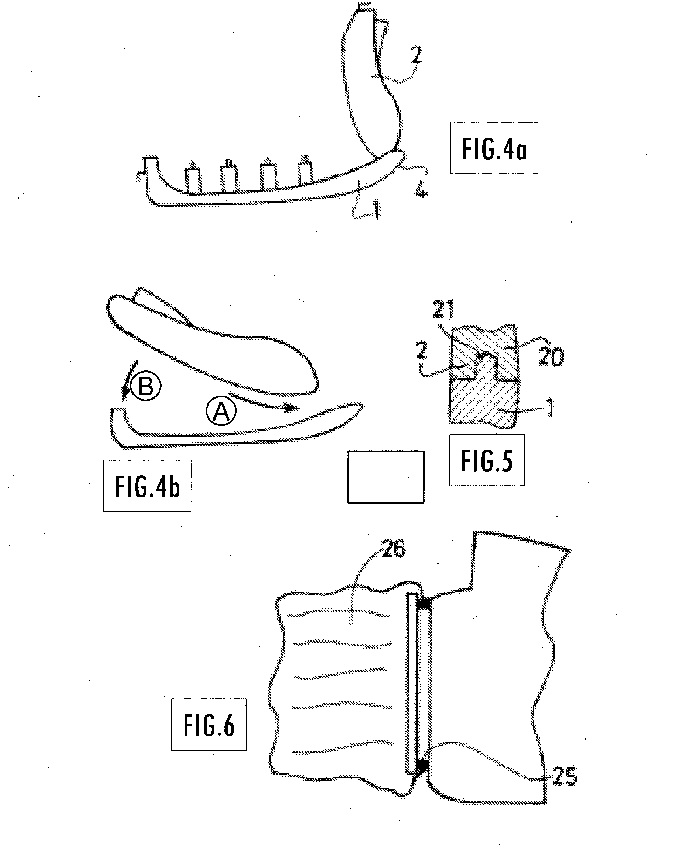

La figure 4 représente .deux vues de l'équipement en position ouverte. La coque inférieure (1) présente à son extrémité (4) un logement permettant de recevoir l'extrémité de la coque supérieure (2) lorsque l'équipement est ouvert (Figure 4a). Cette solution permet de maintenir la coque supérieure (2) sans qu'elle ne vienne en contact avec des surfaces non stériles. Pour fermer la coque il suffit de poser la coque supérieure (2) sur la coque inférieure (1) en deux mouvements successifs (A) et (B) (Figure 4b).

La figure 5 représente une vue de détail de la liaison entre les deux coques. L'une des coques (1) présente sur sa tranche une protubérance périphérique (20) complémentaire d'une nervure périphérique (21) prévue sur la tranche de la coque complémentaire (2), afin d'assurer un chicanage améliorant la fermeture et l'étanchéité.

La figure 6 représente une vue en détail de l'extrémité ouverte de l'équipement. Il présente une gorge annulaire (25) permettant le serrage de l'extrémité d'un manchon souple (26). Ce manchon souple présente une partie au moins en un matériau élastique. Il assure la fermeture étanche autour du membre traité, en une zone éloignée de la plaie ou de la zone traitée.

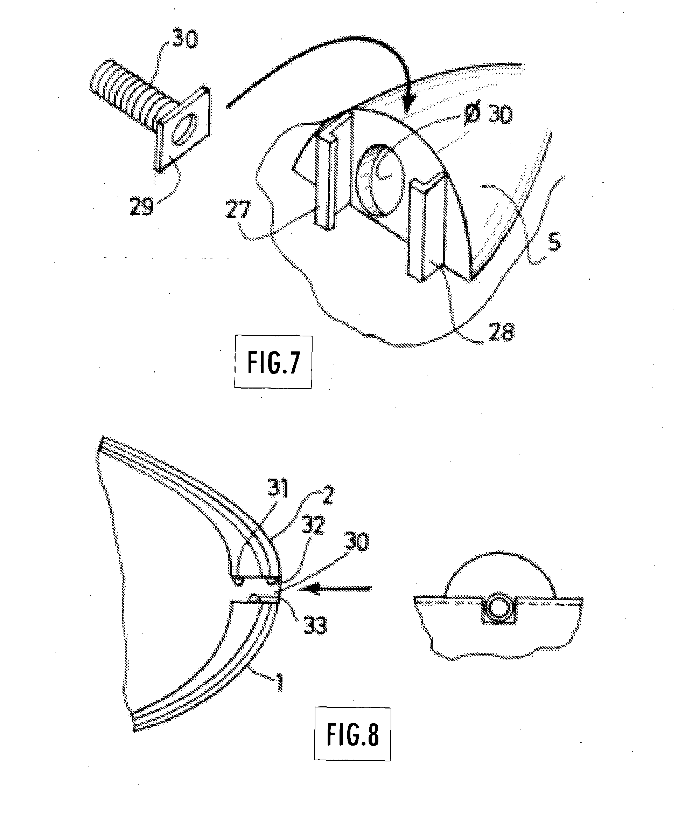

La figure 7 représente une vue de détail d'un exemple de raccordement entre l'entrée de fluide et la coque. La partie saillante (5) présente deux griffes (27, 28) permettant d'introduire la flasque (29) d'un embout (30) sur lequel est monté le tuyau d'arrivée de gaz. Tout autre moyen de connexion d'une source de fluide à la coque qui soit rapide à monter et démonter et étanche peut bien entendu être utilisé dans la présente invention.

La figure 8 représente une vue en coupe de l'extrémité fermée de l'équipement. Les deux coques (1, 2) définissent entre elles une ouverture (30) dont la section correspond sensiblement à un tuyau de sortie. La surface des coques présente des griffes (31 à 33) assurant le maintien du tuyau après que celui-ci a été engagé dans le trou (30).

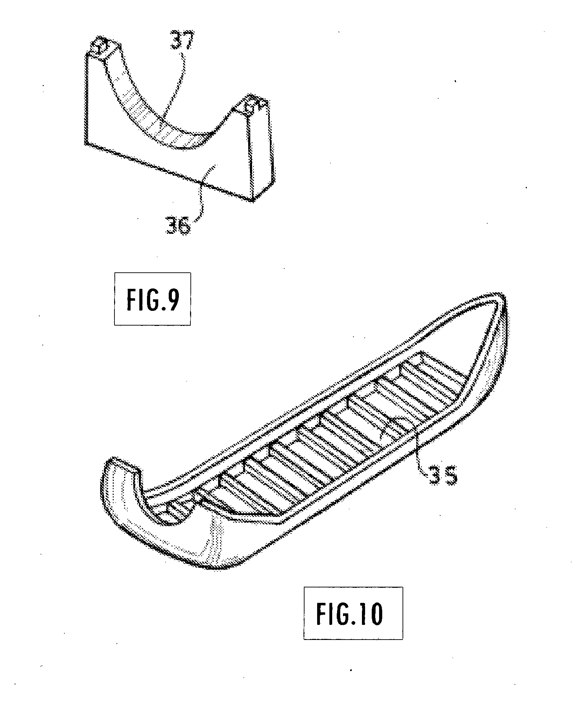

Les figures 9 et 10 représentent des vues d'une variante de réalisation. La coque inférieure (1) présente des encoches (35) pour recevoir des berceaux (36) présentant une découpe en forme de demi-cercle. Ces berceaux sont placés à des intervalles déterminés pour maintenir le membre en évitant les zones blessées.

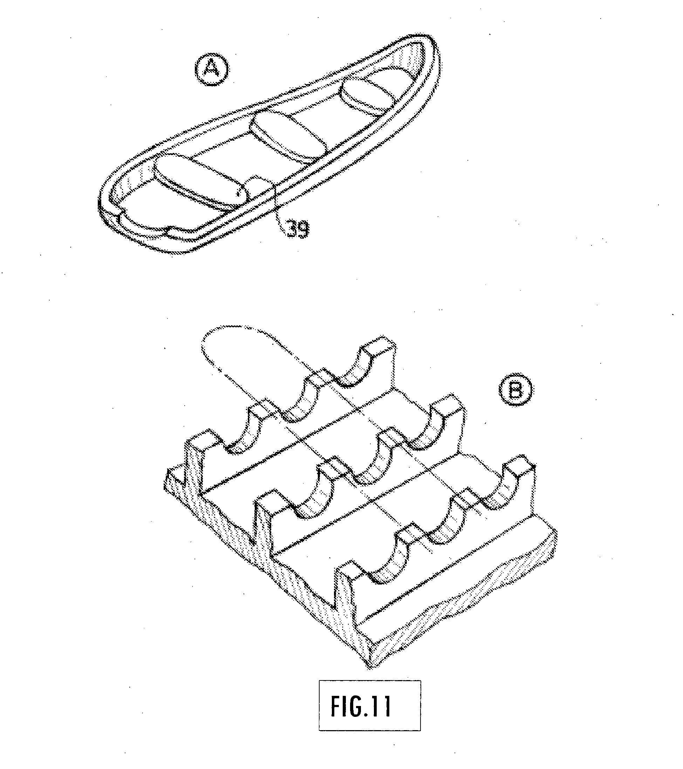

La figure 11 représente une vue d'une autre variante de réalisation. Dans cette solution, le fond de la coque (1) reçoit des coussins gonflables (39) assurant le soutien du membre inséré dans l'enceinte.

Dans le cas de traitements de plus longue durée, il est parfois intéressant de permettre au membre traité de bouger, surtout s'il comprend une articulation. Dans ce cas, l'équipement peut comprendre plusieurs sections adjacentes de l'enveloppe relativement rigides reliées entre elles par des sections souples ou par une charnière pour permettre le débattement d'une articulation de la partie du corps traitée. Une autre solution à ce problème est que l'enveloppe présente un renflement au niveau de l'articulation d'un membre.

L'équipement de la présente invention peut avantageusement être utilisé dans un nombre important de traitements de surface d'une partie du corps, soit dans les domaines thérapeutiques, de soins de santé et « wellness », ou cosmétiques.

Dans le domaine thérapeutique on peut mentionner le traitement de plaies ouvertes, de maladies cutanées, plaies humides telles que par exemple des brûlures, plaies veneuses ou lymphatiques, hypothermie. Par soins de santé on entend ce qui entre généralement dans la classe de médecines alternatives, comprenant des traitements aux huiles, en particulier les huiles essentielles, fleurs de Bach, encens, vapeurs chaudes ou traitements au froid. Enfin, les traitements cosmétiques comprennent une multitudes d'applications, tels le blanchiment des peaux foncées, la pigmentation des peaux claires, la décoloration de la pilosité, le traitement d'éruptions cutanées, la disparition de cicatrices ou de taches, soins hydratants, anti-âge, préparation solaire, etc.

Pour effectuer de tels traitements il suffit d'insérer la partie du corps à traiter entre les au moins deux éléments complémentaires de l'enveloppe telle que décrite plus haut, de relier un point de la cavité formée par ladite enveloppe à une source d'un mélange d'au moins un gaz porteur et d'au moins un principe ayant une activité thérapeutique, de soins de santé ou cosmétique sur la partie du corps traitée et un autre point de ladite cavité à une évacuation des gaz, et injecter dans la cavité enfermant la partie du corps à traiter ledit mélange d'au moins un gaz porteur et d'au moins un principe actif.

Le flux de gaz peut être continu, mais dans certains cas, il peut être discret, remplissant la cavité d'une quantité prédéterminée de gaz et principe actif, puis fermer entrée et sortie de gaz et laisser agir le principe actif sur la partie du corps traitée. Après un temps d'action, la cavité peut être purgée et éventuellement être remplacée par une nouvelle dose de principe actif.

La pression dans la cavité peut varier selon les applications, soit en dessous ou en dessus de, ou à la pression atmosphérique. Il est également possible de faire varier la pression pendant le traitement, par exemple, pour stimuler la circulation ce qui peut augmenter la réceptivité de la partie du corps traitée aux effets des principes actifs utilisés.

## Revendications

1. Equipement constitué par une enveloppe apte à être placée autour d'une partie du corps, ladite enveloppe comprenant :
(a) une arrivée connectable à une source de gaz et une sortie pour l'évacuation des gaz présents à l'intérieur de l'enveloppe ;
(b) au moins deux parties complémentaires (1, 2) définissant entre elles une cavité de section supérieure à celle de la partie du corps à traiter,
**caractérisé en ce qu'**il comprend en outre une alimentation en gaz comprenant un moyen d'enrichissement d'un gaz porteur avec au moins un principe actif, **en ce que** les parois des deux parties complémentaires de l'enveloppe ont une conduction thermique moyenne inférieure ou égale à 65 mW / m·K mesurée selon EN-12667, et **en ce que** les deux parties complémentaires (1, 2) sont constituées de matériau cellulaire ayant subi un traitement de surface sur leurs surfaces interne et externe du type traitement thermique de lissage ou application de peaux ou films de surface de façon à ce que l'enveloppe présente une perméabilité à l'oxygène comprise entre 1 et 300 cm³ / m².atm.24h, de préférence, entre 10 et 200 cm³ / m².atm.24h (ASTM F1927-98).

2. Equipement selon la revendication 1 où les deux parties complémentaires sont des coques (1, 2) présentant une forme générale sensiblement cylindrique, tronconique ou prismatique, dont une ou les deux extrémités comprennent au moins une ouverture munie de moyens permettant de sceller l'équipement autour de la partie du corps traitée.

3. Equipement selon l'une quelconque des revendications précédentes où la conduction thermique est inférieure à 50 mW / m·K, de préférence inférieure à 45 mW / m·K ou, de manière encore préférée, inférieure à 40 mW / m·K.

4. Equipement selon l'une quelconque de revendications précédentes où le matériau cellulaire est à base de polypropylène expansé ou de polystyrène expansé.

5. Equipement selon l'une quelconque des revendications précédentes dans lequel des sections adjacentes de l'enveloppe sont reliées entre elles par des sections souples ou par une charnière pour permettre le débattement d'une articulation de la partie du corps traitée.

6. Equipement selon l'une quelconque des revendications précédentes comprenant en outre des moyens de contrôle d'au moins une des propriétés suivantes : température, hygrométrie, pH, débit de gaz, composition du gaz injecté dans la cavité de l'enveloppe, perte de pression dans la cavité, lesdits moyens servant à maintenir chacune de ces propriétés à des niveaux prédéterminés ou, en cas de déviation excessive de l'une de ces propriétés, à déclencher une alarme ou à arrêter l'équipement.

7. Equipement selon l'une quelconque des revendications précédentes comprenant dans la cavité formée des moyens de soutènement de la partie du corps à traiter.

8. Equipement selon l'une quelconque des revendications précédentes où les deux parties complémentaires (1, 2) comprennent un matériau fibrillaire à base de fibres minérales ou fibres polymériques.

9. Equipement selon l'une quelconque des revendications précédentes pour le traitement thérapeutique de plaies ouvertes, de maladies cutanées, plaies humides telles que par exemple des brûlures, plaies veineuses ou lymphatiques.

## Patentansprüche

1. Ausrüstung, die aus einer Hülle besteht, die dazu ausgelegt ist, um einen Teil des Körpers angeordnet zu werden, wobei die Hülle folgendes umfasst:
(a) einen Zulauf, der mit einer Gasquelle verbunden werden kann, und einen Ausgang für den Austritt der Gase, die im Inneren der Hülle anwesend sind;
(b) mindestens zwei komplementären Teile (1, 2), die untereinander einen Hohlraum mit einem Schnitt definieren, der größer als derjenige des Teils des zu behandelnden Körpers ist,
**dadurch gekennzeichnet, dass** sie aussenden eine Gasversorgung umfasst, umfassend ein Mittel zur Anreicherung eines Trägergases mit mindestens einem Wirkstoff, dadurch, dass die Wände der zwei komplementären Teile der Hülle eine mittlere Wärmeleitfähigkeit von weniger als oder gleich 65 mW/m.K, gemessen gemäß EN-12667, aufweisen, und dadurch, dass die zwei komplementären Teile (1, 2) aus Zellmaterial bestehen, die auf der inneren und äußeren Fläche einer Behandlung vom Typ thermischer Glättebehandlung oder Anwendung von Oberflächenhäuten oder -filmen unterzogen wurden, so dass die Hülle eine Durchlässigkeit für Sauerstoff aufweist, die zwischen 1 und 300 cm³/m².atm.24h, vorzugsweise zwischen 10 und 200 cm³/m².atm.24h (ASTM F1927-98) liegt.

2. Ausrüstung nach Anspruch 1, wobei die zwei komplementären Teile Schalen (1, 2) sind, die eine im Wesentlichen zylindrische, kegelstumpfartige oder prismatische Form aufweisen, wobei das eine oder die zwei Enden mindestens eine Öffnung aufweisen, die mit Mitteln ausgestattet ist, die es ermöglichen, die Ausrüstung um den behandelten Teil des Körpers zu versiegeln.

3. Ausrüstung nach einem der vorhergehenden Ansprüche, wobei die Wärmeleitfähigkeit geringer als 50 mW/m.K, vorzugsweise geringer als 45 mW/m.K oder, noch bevorzugter, geringer als 40 mW/m.K ist.

4. Ausrüstung nach einem der vorhergehenden Ansprüche, wobei das Zellmaterial auf expandiertem Polypropylen oder expandiertem Polystyren basiert.

5. Ausrüstung nach einem der vorhergehenden Ansprüche, wobei benachbarte Abschnitte der Hülle untereinander durch biegsame Abschnitte oder durch ein Scharnier verbunden sind, um den Bewegungsraum eines Gelenks des behandelten Teils des Körpers zu ermöglichen.

6. Ausrüstung nach einem der vorhergehenden Ansprüche, außerdem umfassend Mittel zur Steuerung von mindestens einer der folgenden Eigenschaften: Temperatur, Hygrometrie, pH-Wert, Gasdurchsatz, Zusammensetzung des Gases, das in den Hohlraum der Hülle injiziert wurde, Druckverlust im Hohlraum, wobei die Mittel dazu dienen, jede dieser Eigenschaften auf vorbestimmten Niveaus zu halten, oder für den Fall einer übermäßigen Abweichung einer dieser Eigenschaften, einen Alarm auszulösen oder die Ausrüstung zu stoppen.

7. Ausrüstung nach einem der vorhergehenden Ansprüche, umfassend im gebildeten Hohlraum Mittel zur Stützung des Teils des zu behandelnden Körpers.

8. Ausrüstung nach einem der vorhergehenden Ansprüche, wobei die zwei komplementären Teile (1, 2) ein fibrilläres Material auf der Grundlage von Mineralfasern oder Polymerfasern umfassen.

9. Ausrüstung nach einem der vorhergehenden Ansprüche für die therapeutische Behandlung von offenen Wunden, Hautkrankheiten, feuchten Wunden wie z.B. Verbrennungen, venösen Verletzungen oder lymphatischen Verletzungen.

## Claims

1. Equipment consisting of a jacket suitable for being placed around part of the body, said jacket comprising:
(a) an inlet that can be connected to a source of gas and an outlet for discharging the gases present inside the jacket;
(b) at least complementary parts (1, 2) defining between them a cavity with a cross section greater than that of the part of the body to be treated,
**characterised in that** it further comprises a gas supply comprising a means of enriching a carrier gas with at least one active principle, **in that** the walls of the two complementary parts of the jacket have a mean thermal conduction of less than or equal to 65 mW/m·K measured in accordance with EN 12667, and **in that** the two complementary parts (1, 2) consist of cellular material that has undergone a surface treatment on their internal and external surfaces of the smoothing heat treatment type or application of surface skins or films so that the jacket has a permeability to oxygen of between 1 and 300 cm³/m².atm.24h, preferably between 10 and 200 cm³/m².atm.24h (ASTM F1927-98).

2. Equipment according to claim 1 where the two complementary parts are shells (1, 2) having a substantially cylindrical, frustoconical or prismatic shape overall, one or both ends of which comprise at least one opening provided with means for sealing the equipment around the part of the body being treated.

3. Equipment according to any one of the preceding claims, where the thermal conduction is less than 50 mW/m·K, preferably less than 45 mW/m·K or, even more preferably, less than 40 mW/m·K.

4. Equipment according to any one of the preceding claims, where the cellular material is based on expanded polypropylene or expanded polystyrene.

5. Equipment according to any one of the preceding claims, in which adjacent sections of the jacket are connected together by flexible sections or by a hinge to allow movement of a joint of the part of the body being treated.

6. Equipment according to any one of the preceding claims, further comprising means for controlling at least one of the following properties: temperature, humidity, pH, gas flow rate, composition of the gas injected into the cavity of the jacket and loss of pressure in the cavity, said means serving to maintain each of these properties at predetermined levels or, in the event of excessive deviation of one of these properties, to trigger an alarm or to stop the equipment.

7. Equipment according to any one of the preceding claims, comprising, in the cavity formed, means for supporting the part of the body to be treated.

8. Equipment according to any one of the preceding claims, where the two complementary parts (1, 2) comprise a fibrillar material based on mineral fibres or polymeric fibres.

9. Equipment according to any one of the preceding claims, for the therapeutic treatment of open wounds, skin ailments, moist wounds such as for example burns, or venous or lymphatic wounds.
